# EUROPEAN PATENT SPECIFICATION

(11) **EP 4 514 308 B1**
(45) Date of publication and mention of the grant of the patent: **04.03.2026**
(21) Application number: 23721303.8
(22) Date of filing: 19.04.2023
(51) Int. Cl.: A61K 8/06, A61K 8/31, A61K 8/34, A61K 8/60, A61K 8/73, A61K 8/92, A61Q 1/00, A61Q 1/02, A61Q 1/08, A61Q 1/10

(54) **INVERSE EMULSION COMPRISING A POLYPHENOL, A NON-IONIC POLYSACCHARIDE, A POLYGLYCEROLATED NON-IONIC SURFACTANT WITH AN HLB < 8, A POLYOL, A NON-VOLATILE PLANT OIL, AND A VOLATILE ALKANE**
INVERSE EMULSION MIT EINEM POLYPHENOL, EINEM NICHTIONISCHEN POLYSACCHARID, EINEM POLYGLYCEROLIERTEN NICHTIONISCHEN TENSID MIT EINEM HLB-WERT 8, EINEM POLYOL, EINEM NICHTFLÜCHTIGEN PFLANZENÖL UND EINEM FLÜCHTIGEN ALKAN
ÉMULSION INVERSE COMPRENANT UN POLYPHÉNOL, UN POLYSACCHARIDE NON IONIQUE, UN TENSIOACTIF NON IONIQUE POLYGLYCÉROLÉ AYANT UN HLB < 8, UN POLYOL, UNE HUILE VÉGÉTALE NON VOLATILE ET UN ALCANE VOLATIL

(30) Priority: 28.04.2022 FR 2204004
(43) Date of publication of application: 05.03.2025
(73) Proprietor: L'OREAL, 75008 Paris (FR)
(72) Inventor: DUBUISSON, Pauline, 94550 CHEVILLY LARUE (FR); SOUFFLET, Clémence, 94550 CHEVILLY LARUE (FR); LIAUZUN, Melissa, 94550 CHEVILLY-LARUE (FR); GUILLAUME, Lydie, 94550 CHEVILLY LARUE (FR)
(74) Representative: L'Oreal
(86) International application number: PCT/EP2023/060096
(87) International publication number: WO 2023/208677

(56) References cited:
- FR-A1- 2 773 811
- FR-A1- 2 936 154

## Description

### Technical Field

The present invention is targeted at providing, for the field of the care and/or makeup of keratin materials, in particular the skin, a new composition in the form of an inverse emulsion which is very particularly advantageous with regard to its technical performance qualities and the sensory feelings which it gives the user when it is applied on these keratin materials and in particular on the skin.

Cosmetic compositions, for example foundations, are commonly used to give the skin an esthetic colour, but also to enhance the beauty of irregular skin, by making it possible to hide marks and dyschromias, to reduce the visibility of relief imperfections, such as pores and wrinkles, and to conceal spots and acne marks; in this regard, coverage is one of the main properties sought.

In the cosmetics field, water-in-oil emulsions, also called inverse emulsions, are particularly appreciated by consumers in the field of foundations, sun protection products or moisturizing creams, with regard to their cosmetic properties, in particular with regard to their comfort on application, which is reflected in particular by an absence of or a strong reduction in the feeling of tightness, of drying and/or the tacky and/or greasy effect, in comparison with "direct" oil-in-water emulsions. These same inverse emulsions also make it possible to contain a large amount of fillers and/or pigments and to obtain good homogeneity during application, in comparison with direct emulsions.

At the present time on the market for caring for and making up keratin materials, many products claim persistence throughout the day, withstanding external factors such as water, sebum, mechanical friction, etc.

To provide long-lasting wear for making up the lips and for making up the face, there are known compositions comprising, as coating agent, hydrophobic film-forming polymers such as, for example, silicone resins, for instance the product with the INCI name: Trimethylsiloxysilicate or with the INCI name: Polypropylsilsesquioxane, or silicone acrylate copolymers such as the product with the INCI name: Acrylates/ polytrimethylsiloxymethacrylate copolymer). FR 2 936 154 discloses water-in-oil foundations comprising at least one polyglyceryl polyricinoleate, at least one polyol and at least one volatile linear alkane.

However, consumers, who are increasingly demanding as regards the composition of their cosmetic products, are also seeking to use products with ingredients that are well tolerated such as natural ingredients, with ingredients which have little or no environmental impact and/or ingredients which are compatible with numerous packagings.

There remains the need to find new compositions in the form of a water-in-oil emulsion with ingredients which have little or no environmental impact and/or ingredients which are compatible with numerous packagings, allowing excellent persistence of the expected cosmetic effects, in particular the colour of the makeup on keratin materials, which can range from a day, involving makeup removal at the end of the day, to persistence over several days, which is resistant to mechanical friction, water, sweat and perspiration, sebum, oil, cleansing products such as shower gels, shampoos, two-phase products and certain micellar waters.

Unexpectedly, the inventors have noted that it is possible to achieve these objectives with a composition for caring for and/or making up keratin materials, in particular the skin, in the form of a water-in-oil emulsion comprising, in particular in a physiologically acceptable medium:
a) a continuous oily phase comprising
   i) at least one plant oil; and
   ii) at least one volatile alkane; and
b) at least one aqueous phase dispersed in the oily phase, comprising:
   i) at least one polyphenol X comprising at least two different phenol groups, and
   ii) at least one non-ionic polysaccharide Y, and
   iii) at least one polyol;
c) at least one polyglycerolated non-ionic, preferably non-silicone, surfactant with an HLB of less than or equal to 8.

The inventors have in fact discovered that the compositions of the invention produce excellent persistence of the expected cosmetic effects on the keratin materials which can range from a day, involving makeup removal at the end of the day, to persistence over several days, which is resistant to mechanical friction, water, sebum, oil, cleansing products such as shower gels, shampoos, two-phase products and certain micellar waters. This being due, surprisingly, to the formation of a coating agent resulting from the interaction via hydrogen bonds, at ambient temperature and atmospheric pressure, between the polyphenol X and the non-ionic polysaccharide and also the polyglycerolated surfactant.

This discovery forms the basis of the invention.

### Subjects of the invention

Thus, a first subject of the present invention is a composition for caring for and/or making up keratin materials, in particular the skin, in the form of a water-in-oil emulsion comprising, in particular in a physiologically acceptable medium:
a) a continuous oily phase comprising
   i) at least one plant oil; and
   ii) at least one volatile alkane; and
b) at least one aqueous phase dispersed in the oily phase, comprising:
   i) at least one polyphenol X comprising at least two different phenol groups; and
   ii) at least one non-ionic polysaccharide Y; and
   iii) at least one polyol;
c) at least one polyglycerolated non-ionic, preferably non-silicone, surfactant with an HLB of less than or equal to 8.

According to one preferential embodiment, the composition does not contain any silicone.

A second subject of the present invention relates to a cosmetic process for coating, notably making up, keratin materials, in particular the skin such as the face, the hands, the eyelids, the cheeks, comprising at least: the step of applying the composition, a composition as defined above, to said keratin materials.

### Definitions

In the context of the present invention, the term "keratin material" notably means the skin such as the face, the body, the hands, the cheeks, the eyelids, the contour of the eyes.

The term "physiologically acceptable" is intended to mean compatible with the skin and/or skin appendages, which has a pleasant colour, odour and feel and which does not cause any unacceptable discomfort (stinging or tautness) liable to discourage the consumer from using this composition.

For the purposes of the invention, the term "hydrogen bonding interaction" means an interaction involving a hydrogen atom of one of the two reagents and an electronegative heteroatom of the other reagent, such as oxygen, nitrogen, sulfur and fluorine. In the context of the invention, the hydrogen bonds are formed between the hydroxyl (OH) functions of the reactive phenol groups of the polyphenol X and the reactive hydroxyl groups of the non-ionic polysaccharide Y.

The term "ambient temperature" means 25°C.

The term "atmospheric pressure" means 760 mmHg, i.e. 10⁵ pascals.

For the purposes of the present invention, the term "water-in-oil emulsion", also referred to as inverse emulsion, is understood to denote any composition constituted of a continuous oily phase in which the aqueous phase is dispersed in the form of droplets, so as to observe a mixture which is macroscopically homogeneous to the naked eye.

The term "silicone" is understood to mean any compound comprising at least one silicon atom and in particular at least one Si-O group.

The term "silicone-free composition" is understood to mean any composition containing less than 1.0% by weight of silicone compound, indeed even less than 0.5% by weight, indeed even less than 0.1% by weight, relative to the total weight of the composition, indeed even devoid of silicone compound.

The term "natural compound" refers to any compound derived directly from a plant without having undergone any chemical modification.

The term "compound of natural origin" refers to any compound derived from a plant having undergone a chemical modification.

### Oily phase

A cosmetic composition in accordance with the present invention comprises an oily phase containing i) at least one non-volatile plant oil and ii) at least one volatile alkane.

The term "oil" is understood to mean any fatty substance in the liquid form at ambient temperature (20-25°C) and at atmospheric pressure.

A composition of the invention can comprise an oily phase in a content varying from 5% to 95%, in particular from 10% to 80%, in particular from 15% to 70% and more particularly from 20% to 65% by weight, relative to the total weight of the composition.

### a) Plant oils

The oily phase of the composition of the invention comprises at least one non-volatile plant oil.

The term "non-volatile oil" is understood to mean an oil which remains on the keratin material at ambient temperature and atmospheric pressure for at least several hours and which has in particular a vapour pressure of less than 10⁻³ mmHg (0.13 Pa).

A plant oil can be extracted from a plant product either by a single cold pressing (virgin oil) or by hot pressing and refining (refined oil).

The non-volatile plant oils of the invention are oils extracted directly from plants without undergoing any chemical modification.

Mention may be made, as non-volatile plant oil suitable for the invention, of aloe oil, sweet almond oil, peach kernel oil, peanut oil, argan oil, avocado oil, candlenut oil, baobab oil, borage oil, broccoli oil, calendula oil, camelina oil, carrot oil, safflower oil, hemp oil, rapeseed oil, cottonseed oil, coconut oil, cucumber seed oil, wheat germ oil, jojoba oil, shea oil, alfalfa oil, lily oil, macadamia oil, corn oil, meadowfoam oil, St. John's wort oil, millet oil, monoi oil, hazelnut oil, apricot kernel oil, walnut oil, olive oil, evening primrose oil, barley oil, palm oil, passionflower oil, poppy oil, blackcurrant seed oil, kiwi seed oil, grape seed oil, pistachio oil, pumpkin oil, red kuri squash oil, quinoa oil, musk rose oil, sesame oil, rye oil, soybean oil, sunflower oil, castor oil and watermelon oil, and mixtures thereof.

According to one embodiment, a non-volatile plant oil suitable for the invention can in particular be chosen from macadamia oil, olive oil and mixtures thereof.

A composition according to the invention can comprise from 0.5% to 50% by weight of non-volatile plant oil(s), relative to the total weight of the composition, in particular from 1% to 25% by weight of plant oil and more particularly from 1% to 10% by weight of non-volatile plant oil, relative to the total weight of the composition.

### b) Volatile alkanes

The oily phase of the composition of the invention comprises at least one volatile alkane.

The term "alkane" is understood to mean any compound comprising a saturated, linear or branched, hydrocarbon-based chain constituted exclusively of carbon atoms and hydrogen atoms.

The term "volatile alkane" suitable for the invention is understood to mean a cosmetic alkane which is capable of evaporating on contact with the skin in less than one hour, at ambient temperature (25°C) and atmospheric pressure (760 mmHg, that is to say 101 325 Pa), which is liquid at ambient temperature and which has in particular an evaporation rate ranging from 0.01 to 15 mg/cm²/minute, at ambient temperature (25°C) and atmospheric pressure (760 mmHg).

Mention may be made, by way of example of volatile alkane which can be used in the oily phase of the composition of the invention, of branched alkanes, such as C₈-C₁₆ isoalkanes of petroleum origin (also called isoparaffins), such as

isododecane (also called 2,2,4,4,6-pentamethylheptane), isodecane, isohexadecane, and for example the oils sold under the trade names of Isopars^{®} or Permethyls^{®}. Use will more particularly be made of isododecane.

Mention may also be made, by way of example of volatile alkane which can be used in the oily phase of the composition of the invention, of volatile linear C₆-C₁₄ alkanes.

Mention may be made, by way of example of linear alkanes suitable for the invention, of the alkanes described in the patent applications WO 2007/068371 and WO 2008/155059 of Cognis (mixtures of different alkanes differing by at least one carbon). These alkanes are obtained from fatty alcohols, themselves obtained from copra oil or palm oil.

Mention may be made, by way of example of linear alkanes suitable for the invention, of n-hexane (C₆), n-heptane (C₇), n-octane (C₈), n-nonane (C₉), n-decane (C ₁₀), n-undecane (C₁₁), n-dodecane (C₁₂), n-tridecane (C₁₃), n-tetradecane (C₁₄) and mixtures thereof.

Mention may in particular be made of n-dodecane (C₁₂) and n-tetradecane (C₁₄), which are sold by Sasol respectively under the references Parafol 12-97^{®} and Parafol 14-97^{®}, and also mixtures thereof.

According to one embodiment, a mixture of n-dodecane and n-tetradecane is used. Use may in particular be made of the dodecane/tetradecane mixture in the 85/15 ratio by weight sold by Biosynthis under the reference Vegelight 1214^{©}.

According to yet another embodiment, use is made of a mixture of volatile linear C₉ - C₁₂ alkanes with the INCI name: C9-12 Alkane, such as the product sold by Biosynthis under the reference Vegelight Silk^{©}.

Use will more particularly be made of a mixture of n-undecane (C₁₁) and n-tridecane (C₁₃), such as those obtained in examples 1 and 2 of the application WO2008/155059 from Cognis and such as that sold under the trade name Cetiol Ultimate^{®} by BASF.

Preferably, the volatile alkane(s) are present in a concentration ranging from 0.5% to 90% by weight, in particular from 1% to 50% by weight and more particularly from 5% to 40% by weight, relative to the total weight of the composition.

### c) Additional non-volatile oils

According to a specific embodiment, the oily phase can comprise at least one additional non-volatile hydrocarbon-based oil (different from the non-volatile plant oils described above).

The term "hydrocarbon-based oil" refers to an oil mainly containing carbon and hydrogen atoms and possibly one or more functions chosen from hydroxyl, ester, ether and carboxylic functions.

The term "non-volatile oil" is understood to mean an oil which remains on the keratin material at ambient temperature and atmospheric pressure for at least several hours and which has in particular a vapour pressure of less than 10⁻³ mmHg (0.13 Pa).

Mention may be made, among the additional non-volatile hydrocarbon-based oils which can be used according to the invention, of phytosteryl esters, such as phytosteryl oleate, phytosteryl isostearate and phytosteryl/octyldodecyl lauroyl glutamate (Ajinomoto, Eldew PS203); fatty esters, such as diisopropyl sebacate (Stéarineries Dubois, DUB DIS), isopropyl myristate (Stéarineries Dubois, DUB IPM), isopropyl palmitate (Oleon, Radia 7732); triglycerides constituted of esters of fatty acids and of glycerol, in particular, the fatty acids of which can have chain lengths varying from C₄ to C₃₆ and in particular from C₁₈ to C₃₆, it being possible for these oils to be saturated or unsaturated and linear or branched; these oils can, in particular, be heptanoic or octanoic triglycerides, or also caprylic/capric acid triglycerides, such as those sold by Stéarineries Dubois or those sold under the names Miglyol 810^{®}, 812^{®} and 818^{®} by Dynamit Nobel.

Mention may also be made of squalane, such as the product sold under the trade name Neossance Squalane^{®} by Aprinova.

Mention may also be made of dialkyl ether oils having from 10 to 40 carbon atoms, such as dicaprylyl ether, such as the product sold under the trade name Rofetan OE/ MB^{®} by Ecogreen Oleochemicals or the product sold under the trade name Cetiol OE/ MB^{®} by BASF.

According to a specific form of the invention, dicaprylyl ether will be used.

Preferably, the additional non-volatile hydrocarbon-based oil or oils are present in a concentration of less than or equal to 50% by weight and more particularly from 1% to 25% by weight, relative to the total weight of the composition.

According to a specific embodiment, the ratio by weight of the total amount of plant oil(s) to the total amount of volatile alkane(s) varies from 1/40 to 1/1 and preferably from 1/20 to 1/5.

According to a specific embodiment, the ratio by weight of the total amount of non-volatile plant oil(s) and of additional non-volatile hydrocarbon-based oil(s) to the total amount of volatile alkane(s) varies from 1/8 to 1/1 and preferably from 1/4 to 1/1.

### Aqueous phase

The aqueous phase comprises water and optionally water-soluble or water-miscible ingredients, such as water-soluble solvents.

A water suitable for the invention can be a floral water, such as cornflower water, and/or a mineral water, such as Vittel water, Lucas water or La Roche-Posay water, and/or a thermal water.

Also suitable for the invention may also be a demineralized water.

A composition of the invention can comprise water in a content varying from 5% to 80%, more particularly from 20% to 65% and more preferably still from 20% to 55% by weight, relative to the total weight of the composition.

### Polyphenol X

The polyphenols X that can be used according to the present invention include in their structure at least two different phenol groups.

The term "polyphenol" means any compound having in its chemical structure at least two benzene compounds, in free or fused form, each benzene compound comprising at least one hydroxyl (OH) group, preferably at least 2 hydroxyl groups, or even 3 hydroxyl groups.

The term "different phenol groups" refers to phenol groups that are chemically different.

The polyphenols X that may be used according to the invention may be synthetic or natural. They may be in isolated form or contained in a mixture, notably contained in a plant extract. Polyphenols are phenols comprising at least two phenol groups that are differently substituted on the aromatic ring.

The two classes of polyphenols are flavonoids and non-flavonoids.

Examples of flavonoids that may be mentioned include chalcones such as phloretin, phloridzin, aspalathin or neohesperidin; flavanols such as catechin, fisetin, kaempferol, myricetin, quercetin, rutin, procyanidins, proanthocyanidins, pyroanthocyanidins, theaflavins or thearubigins (or thearubins); dihydroflavonols such as astilbin, dihydro-quercetin (taxifolin) or silibinin; flavanones such as hesperidin, neohesperidin, hesperetin, naringenin or naringin; anthocyanins such as cyanidin, delphinidin, malvidin, peonidin or petunidin; catechin tannins such as tannic acid; isoflavonoids such as daidzein or genistein; neoflavanoids; lignans such as pyroresorcinol; and mixtures thereof.

Among the natural polyphenols X that may be used according to the invention, mention may also be made of lignins.

Examples of non-flavonoids that may be mentioned include curcuminoids such as curcumin or tetrahydrocurcumin; stilbenoids such as astringin, resveratrol or rhaponticin; aurones such as aureusidin; and mixtures thereof.

As polyphenols X that may be used according to the invention, mention may also be made of chlorogenic acid, verbascoside; coumarins substituted with phenols.

According to one particular embodiment of the invention, the polyphenol will be chosen from catechin tannins such as gallotannins chosen from tannic acid; ellagitannins such as epigallocatechin, epigallocatechin gallate, castalagin, vescalagin, vescalin, castalin, casuarictin, castanopsinins, excoecarianins, grandinin, gradinin, roburins, pterocarinin, acutissimin, tellimagrandins, sanguiin, potentillin, pe-dunculagin, geraniin, chebulagic acid, repandusinic acid, ascorgeraniin, stachyurin, ca-suarinin, casuariin, punicacortein, coriariin, cameliatannin, isodehydrodigalloyl, dehy-drodigalloyl, hellinoyl, punicalagin and rhoipteleanins.

According to one particular embodiment of the invention, the polyphenol X is epigallocatechin, in particular a green tea extract having the INCI name Green Tea Extract, notably comprising at least 45% epigallocatechin relative to the total weight of said extract, for instance the commercial product sold under the name Dermofeel Phenon 90 M-C^{®} sold by Evonik Nutrition & Care or the commercial product sold under the name Tea Polyphenols Green Tea Extract^{®} by Tayo Green Power.

According to one particular embodiment of the invention, the polyphenol X is a pro-cyanidin or a mixture of procyanidins, in particular an extract of maritime pine bark having the INCI name *Pinus pinaster* Bark/Bud Extract, notably comprising at least 65% by weight of procyanidins relative to the total weight of said extract, such as the commercial product sold under the name Pycnogenol^{®} sold by Biolandes Arômes.

Use will more particularly be made, as polyphenol X, of tannic acid, such as the commercial product sold under the name Brewtan F^{®} by Anijomoto Omnichem NV.

According to one particular embodiment, the polyphenol(s) X of the invention is (are) present in an amount of greater than or equal to 0.8% by weight, preferably greater than or equal to 1.0% by weight and more particularly greater than or equal to 2.0% by weight relative to the total weight of the composition.

According to one particular embodiment, the polyphenol(s) X of the invention is (are) present in an amount ranging from 1.0% to 30.0% by weight, and more particularly ranging from 2.0% to 30% by weight relative to the total weight of the composition.

### Non-ionic polysaccharide Y

The non-ionic polysaccharides Y are carbohydrate macromolecules formed by the linking of a large number of hydrophilic elementary sugars (saccharides) bonded together via O-oside bonds.

According to one particular embodiment, the non-ionic polysaccharides in accordance with the invention will be different from sugars derived from fruits or vegetables, in particular simple sugars such as glucose, sucrose, fructose and sorbitol.

They may be chosen from native polysaccharides, modified polysaccharides, and mixtures thereof.

The term "modified polysaccharide" means any chemically or enzymatically modified polysaccharide including at least one functional group.

The functional groups may be hydrocarbon-based groups (constituted essentially of carbon and hydrogen atoms) such as alkyl, alkenyl, aryl (i.e. phenyl) or aralkyl (i.e. benzyl) groups. The hydrocarbon-based groups may be unsubstituted, for example constituted of a simple alkyl chain, or may be substituted with groups, for instance aromatic groups such as aryl (i.e. phenyl) or aralkyl (i.e. benzyl) groups or alternatively polar groups, for instance hydroxyls.

In one preferred embodiment, the molar mass of compound Y is greater than 2000 g/ mol, or even greater than 3500 g/mol.

The optionally modified non-ionic polysaccharides that are suitable for use in the invention may be homopolysaccharides such as fructans, glucans, galactans and mannans or heteropolysaccharides such as hemicellulose. They may be starchy polysaccharides such as native or modified starches. The non-starchy polysaccharides may be chosen from polysaccharides produced by microorganisms; polysaccharides isolated from algae, and higher plant polysaccharides, such as homogeneous polysaccharides, in particular celluloses and derivatives thereof or fructoses, heterogeneous polysaccharides such as galactomannans, glucomannans and pectins, and derivatives thereof; and mixtures thereof. In particular, the polysaccharides may be chosen from fructans, glucans, amylose, amylopectin, glycogen, pullulan, dextrans, celluloses and derivatives thereof, in particular methylcelluloses, hydroxyalkylcelluloses and ethylhy-droxyethylcelluloses, cetylhydroxyethylcelluloses, mannans, xylans, arabans, galactans, galacturonans, chitin, chitosans, glucuronoxylans, arabinoxylans, xy-loglucans, glucomannans, arabinogalactans, agars, karaya gums (about 40% acid), locust bean gums, guar gums and non-ionic derivatives thereof, in particular hydroxypropyl guar, and biopolysaccharide gums of microbial origin, in particular scle-roglucan gums. They are notably chosen from celluloses such as cetylhydroxyethylcelluloses; guar gums that are notably modified such as hydroxypropyl guars, agarose; pullulans, starches, inulins and starches.

According to a preferential embodiment, the polysaccharide(s) Y will be chosen from pullulans; celluloses such as cetylhydroxyethylcellulose; modified guar gums, in particular hydroxypropyl guar; and mixtures thereof.

According to one preferential embodiment, the polysaccharide Y is a pullulan.

The pullulan in accordance with the invention is a polysaccharide constituted of maltotriose units, known under the name α-(1,4)-α(1,6)-glucan. Three glucose units in maltotriose are connected via an α-(1,4) glycoside bond, whereas the consecutive maltotriose units are connected to each other via an α-(1,6) glycoside bond. It is produced from starch by the fungus *Aureobasidium pullulans.* The pullulan is for example produced under the trade reference Pullulan PF 20^{®} by the Hayashibara group in Japan or under the reference Aqua Beta^{®} by Daiso Co. Ltd., such as the product sold under the name Pullulan Cosmetic Grade^{®} by Hayashibara.

According to one preferential embodiment, the compound(s) Y of the invention is (are) present in an amount greater than or equal to 0.8% by weight, preferably greater than or equal to 1.0% by weight, in particular greater than or equal to 2.0% by weight, more particularly in an amount ranging from 2% to 10% by weight, relative to the total weight of the composition (A).

According to one preferential embodiment of the invention, the mole ratio of the reactive hydroxyl (OH) groups of the polyphenol(s) X to the reactive hydroxyl groups of the non-ionic polysaccharide(s) Y capable of forming hydrogen bonds with said hydroxyl groups preferentially ranges from 1/3 to 20, more preferentially from 1/2 to 15 and more particularly from 3/4 to 3.

### Polyglycerolated emulsifying surfactant with an HLB ≤ 8

The composition according to the invention comprises at least one polyglycerolated emulsifying non-ionic surfactant with an HLB ≤ 8.

For the purposes of the present invention, a "polyglycerolated compound" is understood to mean any molecule comprising in its chemical structure at least two glycerol groups or a chain comprising -(O-CH₂-CHOH-CH₂) glycerol units.

For the purposes of the present invention, the term "surfactant" is understood to mean an amphiphilic compound, that is to say a compound exhibiting two parts of different polarities. Generally, one is lipophilic (soluble or dispersible in an oily phase). The other is hydrophilic (soluble or dispersible in water). Emulsifying surfactants are characterized by the value of their HLB (Hydrophilic Lipophilic Balance), the HLB being the ratio of the hydrophilic part to the lipophilic part in the molecule. The term "HLB" is well known to those skilled in the art and is described, for example, in "The HLB System. A Time-Saving Guide to Emulsifier Selection" (published by ICI Americas Inc; 1984). For the emulsifying surfactants used in the preparation of water-in-oil emulsions, their HLB value is generally less than or equal to 8 and more particularly ranges from 3 to 8. The value of the HLB can be determined by the Griffin method or the Davies method.

Among the polyglycerolated emulsifying non-ionic surfactants with an HLB ≤ 8 that can be used according to the invention, mention may be made notably of
- esters of isostearic acid and of a polyglycerol containing from 2 to 10 mol of glycerol units, such as for example Polyglyceryl-4 Isostearate sold under the name Isolan GI34^{®} by Evonik Nutrition & Care GmbH; Polyglyceryl-3 Diisostearate sold under the name Lameform TGI^{®} by Cognis; Polyglyceryl-2 Diisostearate sold under the name Emalex PGSA^{®} by Nihon Emulsion; Polyglyceryl-10 Isostearate sold under the name Nikkol Decaglyn 1-IS^{®} by Nihon Surfactant; Polyglyceryl-4 Diisostearate/ Polyhydroxystearate Sebacate sold under the name Isolan GPS^{®} by Evonik Nutrition & Care GmbH; Polyglyceryl-2 Triisostearate sold under the name Cithrol PG23IS^{®} by Croda Europe, Ltd;
- esters of stearic acid and of a polyglycerol containing 2 or 3 mol of glycerol units, such as Polyglyceryl-2 Sesquistearate sold by Taiyo Kagaku Company under the name Sunsoft Q-18B^{®}, Polyglyceryl-3 Distearate sold by BASF under the name Cremophor GS 32^{®} , Polyglyceryl-2 Stearate sold under the name Hostacerin DGMS^{®} by Clariant International Ltd;
- esters of oleic acid and of a polyglycerol containing 2 or 3 mol of glycerol units, such as Polyglyceryl-2 Oleate sold under the name Nikkol DGMO-CV^{®} by Nikko Chemicals, Polyglyceryl-3 Oleate sold under the name Isolan GO 33^{®} by Evonik Nutrition & Care GmbH, Polyglyceryl-2 Dioleate; Polyglyceryl-3 Dioleate sold under the name Plurol Oleique CC 497^{®} by Gattefosse;
- polyglyceryl polyricinoleate(s) containing from 3 to 6 mol of glycerol units, such as Polyglyceryl-3 Polyricinoleate, notably sold by Karlshamns under the name Akoline PGPR^{®} or by Stéarinerie Dubois Fils under the name Dub PGPR or by Dr. Straetmans under the name Dermofeel^{®} or by Croda under the name Crester PR^{®} or also by Sasol under the name Imwitor 600^{®}; the Polyglyceryl-5 Polyricinoleate sold by Taiyo Kagaku Co. Ltd under the name Sunsoft No.818R^{®}; the Polyglyceryl-6 Polyricinoleate sold by Nikko Chemicals Co. Ltd under the name Hexaglyn PR-15^{®} or by Sakamoto Yakuhin Kogyo Co. Ltd under the name SY Glyster CRS-75^{®}.

According to one embodiment, mixtures of these compounds can be employed.

More particularly, the polyglycerolated non-ionic surfactant will be chosen from polyglyceryl polyricinoleate(s) containing from 3 to 6 mol of glycerol units, and more particularly the Polyglyceryl-6 Polyricinoleate sold by Nikko Chemicals Co. Ltd under the name Hexaglyn PR-15^{®} or by Sakamoto Yakuhin Kogyo Co. Ltd under the name SY-Glyster CRS-75^{®}.

A composition of the invention preferably comprises from 2% to 10% by weight of polyglycerolated non-ionic surfactant(s) with an HLB ≤ 8, in particular from 2% to 7% by weight, relative to the total weight of the composition.

### Polyol

The composition according to the invention comprises at least one polyol.

For the purposes of the present invention, the term "polyol" should be understood as meaning any organic molecule including at least two free hydroxyl groups.

The polyol(s) in accordance with the present invention are present in liquid form at ambient temperature (20-25°C).

A polyol that is suitable for use in the invention may be a compound of linear, branched or cyclic, saturated or unsaturated alkyl type, bearing on the alkyl chain at least two -OH functions, in particular at least three -OH functions and more particularly at least four -OH functions.

The polyols that are advantageously suitable for formulating a composition according to the present invention are those notably having 2 to 4 carbon atoms.

Advantageously, the polyol may be chosen from C₃-C₄ alkanediols, glycerol, and mixtures thereof.

The term "C₃-C₄ alkanediol" is understood to mean any saturated, linear or branched, alkane compound comprising a hydrocarbon-based chain comprising exclusively hydrogen atoms, 3 or 4 carbon atoms and two hydroxyl groups at different positions.

The C₃-C₄ alkanediols can be chosen from 2-methyl-1,3-propanediol (INCI name Methyl Propanediol), 1,2-propanediol (INCI name Propylene Glycol), 1,3-propanediol (INCI name Propanediol), 1,2-butanediol (or 1,2-butylene glycol), 1,4-butanediol (or tetramethylene glycol), 2,3-butanediol (or 2,3-butylene glycol) or 1,3-butanediol (INCI name Butylene Glycol).

According to one preferred embodiment, the composition of the invention comprises a mixture of C₃-C₄ alkanediol(s) and of glycerol.

According to one preferred embodiment of the invention, said polyol is chosen from propanediol, glycerol, and mixtures thereof.

According to one preferred embodiment of the invention, said polyol is chosen from a mixture of propanediol and of glycerol.

A composition of the invention preferably comprises from 0.1% to 25% by weight total amount of polyol(s), in particular from 1% to 15% by weight and more particularly from 1% to 10% by weight relative to the total weight of the composition.

### Monoalcohol

According to one particular embodiment of the invention, the composition according to the invention also comprises at least one monoalcohol comprising from 2 to 8 carbon atoms, notably from 2 to 6 carbon atoms and in particular from 2 to 4 carbon atoms such as ethanol, isopropanol, propanol or butanol, and mixtures thereof, and more particularly ethanol.

The monoalcohol(s) comprising from 2 to 8 carbon atoms is (are) preferably present in contents of from 2% to 10% relative to the total weight of the composition.

### Sugar alcohol

According to a specific embodiment of the invention, the composition according to the invention additionally comprises at least one sugar alcohol.

The term "sugar alcohol" denotes a sugar comprising only hydroxyl functions. These sugar alcohols differ from ketoses and aldoses, which comprise ketone or aldehyde functions. Sugar alcohols are in particular described in the encyclopedia Kirk-Othmer Encyclopedia of Chemical Technology, John Wiley and Sons, "Sugar Alcohols" article, 2005.

Preferably, the sugar alcohols which can be used in the compositions according to the invention correspond to the general formula:

[Chem.4] HO-CH₂-(CHOH)ₙ-CH₂-OH

with n an integer ranging from 2 to 5.

Preferentially, the sugar alcohol is chosen from erythritol, sorbitol, threitol, ribitol, arabinitol, allitol, dulcitol, iditol, altritol, lactitol, maltitol, mannitol, xylitol and the mixtures of these compounds.

Particularly preferably, the sugar alcohol is sorbitol (INCI names: Sorbitol or Hydrogenated Starch Hydrolysate).

The composition according to the invention preferably contains from 1% to 15% by weight of sugar alcohol(s), more preferentially still from 2% to 12% by weight, even better still from 4% to 10% by weight, relative to the total weight of the composition.

### Monoester of saturated and linear C₇-C₁₄ carboxylic acid and of glycerol

According to a specific embodiment of the invention, the composition according to the invention additionally comprises at least one monoester of saturated and linear C₇ - C₁₄ carboxylic acid and of glycerol.

Mention may be made, among monoesters of saturated and linear C₇-C₁₄ carboxylic acid and of glycerol, which can be used in the compositions of the invention, of:
- Glyceryl Heptanoate (C₇), such as the product with the trade name Lexgard Natural GH70^{®} (Inolex Inc.);
- Glyceryl Caprylate (C₈), such as products with the trade name AEC Glyceryl Caprylate^{®} (A & E Connock Perfumery & Cosmetics); Capmul 708G^{©} (Abitec Corporation); Cremercoor GC 8^{®} (IOI Oleo GmbH); Dermosoft GMCY^{®} (Dr. Straetmans GmbH); DUB 8G^{®} (Stéarinerie Dubois Fils); Imwitor 308^{®} and Imwitor 988^{®} (IOI Oleo GmbH); Lexgard GMCY MB^{®} (Inolex Inc.); Oristar GCC (Orient Stars LLC); Sunsoft 707^{®} and Sunsoft 700 P-2^{®} (Taiyo Kagaku Company Ltd.) ; 104528 Symlite G8^{®} (Symrise);
- Glyceryl Caprylate (C₈)/Caprate(C₁₀), such as products with the trade name AEC Glyceryl Caprylate/Caprate (A & E Connock Perfumery & Cosmetics Ltd) ; Cremercoor GC810 (IOI Oleo GmbH); Dub 810 G (Stéarinerie Dubois Fils);
- Glyceryl Caprate (C₁₀), such as products with the trade name Capmul MCM C-10^{®} (Abitec Corporation); Dermosoft GMC^{®} (Dr. Straetmans GmbH); AEC Glyceryl Caprate^{®} (A & E Connock Perfumery & Cosmetics); Lexgard GMC^{®} (Inolex Inc.); Sunsoft 760 (Taiyo Kagaku Company Ltd) ;
- Glyceryl Laurate (C₁₂), such as the products with the trade name AEC Glyceryl Laurate^{®} (A & E Connock Perfumery & Cosmetics); Colonial Monolaurin^{®} (Colonial Chemical Inc.); DUB LG^{®} (Stéarinerie Dubois Fils); Jeechem MLD^{®} (Jeen International Corporation); Lauricidin (Med-Chem Labs Inc.) Monomuls 90-L 12 (BASF Corporation); Oristar GL (Orient Stars LLC); Pelemol GMLA^{®} (Phoenix Chemical Inc.); Protachem MLD^{®} (Protameen Chemicals); Sunsoft 750^{®} (Taiyo Kagaku Company Ltd) ; Ultrapure GML^{®} (Ultra Chemical, Inc.)
- Glyceryl Myristate (C₁₄), such as products with the trade name AEC Glyceryl Myristate^{®} (A & E Connock Perfumery & Cosmetics); Cithrol GMM (Croda Europe); DUB MG (Stéarinerie Dubois Fils); Nikkol MGM (Nikko Chemicals Co. Ltd) ; Oristar GMA (Orient Stars LLC); Sunsoft 8002 (Taiyo Kagaku Company Ltd) and
- mixtures thereof.

According to a specific embodiment of the invention, Glyceryl Caprylate will be used.

The composition according to the invention preferably contains from 0.05% to 3% by weight of monoester(s) of saturated and linear C₇-C₁₄ carboxylic acid and of glycerol, more preferentially still from 0.1% to 2% by weight, better still from 0.2% to 1% by weight, relative to the total weight of the composition.

### Pulverulent colorant

According to one particular embodiment of the invention, the composition also comprises at least one pulverulent colorant.

The pulverulent colorants can be chosen from pigments, nacres and mixtures thereof.

The term "pigments" means white or coloured, mineral or organic particles, which are insoluble in an aqueous medium, and which are intended to colour and/or opacify the resulting composition and/or deposit.

According to a specific embodiment, the pigments used according to the invention are chosen from mineral pigments.

The term "mineral pigment" is understood to mean any pigment which satisfies the definition of Ullmann's Encyclopedia in the chapter "Pigments, Inorganic". Among the mineral pigments that are useful in the present invention, mention may be made of zirconium oxide or cerium oxide, and also zinc oxide, iron oxide (black, yellow or red) or chromium oxide, manganese violet, ultramarine blue, chromium hydrate and ferric blue, titanium dioxide, and metal powders, for instance aluminum powder and copper powder. The following mineral pigments may also be used: Ta₂O₅, Ti₃O₅, Ti₂O₃, TiO, ZrO₂ as a mixture with TiO₂, ZrO₂, Nb₂O₅, CeO₂ or ZnS.

The size of the pigment that is useful in the context of the present invention is generally greater than 100 nm and may range up to 10 µm, preferably from 200 nm to 5 µm and more preferentially from 300 nm to 1 µm.

According to a particular form of the invention, the pigments have a size characterized by a D[50] greater than 100 nm and possibly ranging up to 10 µm, preferably from 200 nm to 5 µm and more preferentially from 300 nm to 1 µm.

The sizes are measured by static light scattering using a commercial MasterSizer 3000^{®} particle size analyzer from Malvern, which makes it possible to determine the particle size distribution of all of the particles over a wide range which may extend from 0.01 µm to 1000 µm. The data are processed on the basis of the standard Mie scattering theory. This theory is the most suitable for size distributions ranging from submicron to multimicron; it allows an "effective" particle diameter to be determined. This theory is described in particular in the publication by Van de Hulst, H.C., Light Scattering by Small Particles, Chapters 9 and 10, Wiley, New York, 1957.

D[50] represents the maximum size exhibited by 50% by volume of the particles.

In the context of the present invention, the mineral pigments are more particularly iron oxide and/or titanium dioxide. By way of example, mention may be made more particularly of titanium dioxides and iron oxide coated with aluminium stearoyl glutamate, sold, for example, under the reference NAI^{®} by Miyoshi Kasei.

As mineral pigments that may be used in the invention, mention may also be made of nacres.

The term "nacres" should be understood as meaning coloured particles of any form, which may or may not be iridescent, notably produced by certain molluscs in their shell, or alternatively synthesized, and which have a colour effect via optical interference.

The nacres may be chosen from nacreous pigments such as titanium mica coated with an iron oxide, titanium mica coated with bismuth oxychloride, titanium mica coated with chromium oxide, titanium mica coated with an organic dye and also nacreous pigments based on bismuth oxychloride. They may also be mica particles, at the surface of which are superposed at least two successive layers of metal oxides and/or of organic colorants.

According to a specific embodiment, the pigments used according to the invention are chosen from mineral pigments.

Examples of nacres that may also be mentioned include natural mica covered with titanium oxide, with iron oxide, with natural pigment or with bismuth oxychloride.

The nacres can more particularly have a yellow, pink, red, bronze, orangey, brown, gold and/or coppery colour or tint.

Among the pigments that may be used according to the invention, mention may also be made of those having an optical effect different from a simple conventional colouring effect, i.e. a unified and stabilized effect such as produced by conventional colorants, for instance monochromatic pigments. For the purposes of the invention, the term "stabilized" means lacking the effect of variability of the colour with the angle of observation or in response to a temperature change.

For example, this material may be chosen from particles with a metallic tint, go-niochromatic colouring agents, diffractive pigments, thermochromic agents, optical brighteners, and also fibres, notably interference fibres. Needless to say, these various materials may be combined in order simultaneously to afford two effects, or even a novel effect in accordance with the invention.

According to one particular embodiment, the composition (B) according to the invention comprises at least one uncoated pigment.

According to another specific embodiment, the composition (B) according to the invention comprises at least one pigment coated by at least one lipophilic or hydrophobic compound.

This type of pigment is particularly advantageous. Insofar as they are treated with a hydrophobic compound, they show predominant affinity for an oily phase, which can then convey them.

The coating may also comprise at least one additional non-lipophilic compound.

For the purposes of the invention, the "coating" of a pigment according to the invention generally denotes the total or partial surface treatment of the pigment with a surface agent, absorbed on, adsorbed on or grafted to said pigment.

The surface-treated pigments may be prepared according to surface treatment techniques of chemical, electronic, mechanochemical or mechanical nature that are well known to those skilled in the art. Commercial products may also be used.

The surface agent may be absorbed, adsorbed or grafted onto the pigments by evaporation of solvent, chemical reaction and creation of a covalent bond.

According to one variant, the surface treatment consists in coating the pigments.

The coating can represent from 0.1% to 20% by weight and in particular from

0.5% to 5% by weight of the total weight of the coated pigment.

The coating may be produced, for example, by adsorption of a liquid surface agent onto the surface of the solid particles by simple mixing with stirring of the particles and of said surface agent, optionally with heating, prior to the incorporation of the particles into the other ingredients of the makeup or care composition.

The coating may be produced, for example, by chemical reaction of a surface agent with the surface of the solid pigment particles and creation of a covalent bond between the surface agent and the particles. This method is notably described in patent US 4 578 266.

The chemical surface treatment may consist in diluting the surface agent in a volatile solvent, dispersing the pigments in this mixture and then slowly evaporating off the volatile solvent, so that the surface agent is deposited on the surface of the pigments.

When the pigment comprises a lipophilic or hydrophobic coating, the latter is preferably present in the fatty phase of the composition according to the invention.

According to one particular embodiment of the invention, the pigments may be coated according to the invention with at least one compound chosen from silicone surface agents; fluoro surface agents; fluorosilicone surface agents; metal soaps; N-acylamino acids or salts thereof; lecithin and derivatives thereof; isopropyl triisostearyl titanate; isostearyl sebacate; natural plant or animal waxes; polar synthetic waxes; fatty esters; phospholipids; and mixtures thereof.

According to one particular embodiment of the invention, the pigments may be coated with a hydrophilic compound.

According to one particular embodiment, the colorant is an organic pigment, which is synthetic, natural or of natural origin.

The term "organic pigment" refers to any pigment that satisfies the definition in Ullmann's encyclopedia in the chapter on organic pigments. The organic pigment may notably be chosen from nitroso, nitro, azo, xanthene, quinoline, anthraquinone, phthalocyanine, metal-complex type, isoindolinone, isoindoline, quinacridone, perinone, perylene, diketopyrrolopyrrole, thioindigo, dioxazine, triphenylmethane or quinophthalone compounds.

The organic pigment(s) may be chosen, for example, from carmine, carbon black, aniline black, melanin, azo yellow, quinacridone, phthalocyanine blue, sorghum red, the blue pigments codified in the Color Index under the references CI 42090, 69800, 69825, 73000, 74100 and 74160, the yellow pigments codified in the Color Index under the references CI 11680, 11710, 15985, 19140, 20040, 21100, 21108, 47000 and 47005, the green pigments codified in the Color Index under the references CI 61565, 61570 and 74260, the orange pigments codified in the Color Index under the references CI 11725, 15510, 45370 and 71105, the red pigments codified in the Color Index under the references CI 12085, 12120, 12370, 12420, 12490, 14700, 15525, 15580, 15620, 15630, 15800, 15850, 15865, 15880, 17200, 26100, 45380, 45410, 58000, 73360, 73915 and 75470, and the pigments obtained by oxidative polymerization of indole or phenol derivatives as described in patent FR 2 679 771.

The pigments may also be in the form of composite pigments as described in patent EP 1 184 426. These composite pigments may notably be composed of particles including a mineral core at least partially covered with an organic pigment and at least one binder for fixing the organic pigments to the core.

The pigment may also be a lake. The term "lake" means insolubilized dyes adsorbed onto insoluble particles, the assembly thus obtained remaining insoluble during use.

The mineral substrates onto which the dyes are adsorbed are, for example, alumina, silica, calcium sodium borosilicate or calcium aluminium borosilicate and aluminium.

Among the organic dyes, mention may be made of cochineal carmine. Mention may also be made of the products known under the following names: D&C Red 21 (CI45 380), D&C Orange 5 (CI 45 370), D&C Red 27 (CI 45 410), D&C Orange 10 (CI 45 425), D&C Red 3 (CI 45 430), D&C Red 4 (CI 15 510), D&C Red 33 (CI 17 200), D&C Yellow 5 (CI 19 140), D&C Yellow 6 (CI 15 985), D&C Green 5 (CI 61 570), D&C Yellow 10 (CI 77 002), D&C Green 3 (CI 42 053), D&C Blue 1 (CI 42 090).

An example of a lake that may be mentioned is the product known under the name D&C Red 7 (CI 15 850:1).

### Cosmetic compositions

### Cosmetic compositions

The present invention also relates to a cosmetic composition comprising, in a physiologically acceptable medium, a composition as defined above.

The physiologically acceptable medium is generally adapted to the nature of the support onto which the composition has to be applied, and also to the appearance under which the composition has to be packaged.

The compositions according to the invention can additionally comprise additives commonly used in care and/or makeup products, such as:
- active agents, such as vitamins, for example vitamins A, E, C or B3;
- sunscreens;
- additional colorants;
- fillers;
- lipophilic gelling agents;
- fragrances;
- preserving agents;
- and mixtures thereof.

It is a matter of routine operations for those skilled in the art to adjust the nature and the amount of the additives present in the compositions in accordance with the invention so that the cosmetic properties desired for these are not thereby affected.

### Additional colorants

A composition according to the invention can additionally comprise at least one additional water-soluble or fat-soluble colorant and preferably in a proportion of at least 0.01% by weight, relative to the total weight of the composition.

For obvious reasons, this amount is liable to vary significantly with regard to the desired intensity of the colour effect and to the colour intensity provided by the colorants under consideration, and its adjustment clearly falls within the competence of those skilled in the art.

The additional colorants suitable for the invention can be fat-soluble.

For the purposes of the invention, the term "liposoluble colorant" means any natural or synthetic, generally organic compound, which is soluble in an oily phase or in solvents that are miscible with a fatty substance, and which is capable of imparting colour.

Mention may in particular be made, as fat-soluble dyes suitable for the invention, of synthetic or natural fat-soluble dyes, such as, for example, DC Red 17, DC Red 21, DC Red 27, DC Green 6, DC Yellow 11, DC Violet 2, DC Orange 5, Sudan red, carotenes (β-carotene, lycopene), xanthophylls (capsanthin, capsorubin, lutein), palm oil, Sudan brown, quinoline yellow, annatto or curcumin.

The additional colorants suitable for the invention can be water-soluble.

For the purposes of the invention, the term "water-soluble colorant" means any natural or synthetic, generally organic compound, which is soluble in an aqueous phase or water-miscible solvents and which is capable of imparting colour.

As water-soluble dyes that are suitable for use in the invention, mention may be made notably of synthetic or natural water-soluble dyes, for instance FDC Red 4, DC Red 6, DC Red 22, DC Red 28, DC Red 30, DC Red 33, DC Orange 4, DC Yellow 5, DC Yellow 6, DC Yellow 8, FDC Green 3, DC Green 5, FDC Blue 1, betanine (beetroot), carmine, copper chlorophyllin, methylene blue, anthocyanins (enocianin, black carrot, hibiscus, elder), caramel and riboflavin.

### Fillers

The compositions in accordance with the invention can also comprise at least one filler which makes it possible in particular to confer on them additional properties of improved stability, wear property, coverage and/or mattness.

The term "filler" should be understood as meaning colourless or white solid particles of any shape which are provided in an insoluble form and dispersed in the medium of the composition. These particles, of mineral or organic nature, make it possible to confer body or firmness on the composition and/or softness and uniformity on the makeup.

The fillers can be inorganic or organic.

Preferably, they will be chosen from natural fillers or fillers of natural origin.

The fillers used in the compositions according to the present invention can be of lamellar, globular, spherical or fibrous forms or of any other form intermediate between these defined forms.

The fillers according to the invention may or may not be surface-coated, and in particular they may be surface-treated with silicones, amino acids, fluorinated derivatives or any other substance which promotes the dispersion and the compatibility of the filler in the composition.

Mention may be made, as examples of inorganic fillers, of talcs, natural or synthetic micas, such as synthetic fluorphlogopites, silica, hollow silica microspheres, kaolin, calcium carbonate, magnesium carbonate, hydroxyapatite, boron nitride, glass or ceramic microcapsules, or composites of silica and of titanium dioxide, such as the TSG^{®} series sold by Nippon Sheet Glass.

According to one particular embodiment, use will be made of an inorganic filler chosen from natural or synthetic micas, calcium carbonate and mixtures thereof.

Mention may be made, as examples of organic fillers, of micronized natural waxes; metal soaps derived from organic carboxylic acids having from 8 to 22 carbon atoms, preferably from 12 to 18 carbon atoms, for example zinc, magnesium or lithium stearate, zinc laurate or magnesium myristate; lauroyl lysine; or cellulose powders, such as that sold by Daito in the Cellulobeads^{®} range.

Preferably, the filler(s) are present in the composition in a content ranging from 0.5% to 20% by weight, preferably from 1% to 15% by weight, more particularly from 3% to 10% by weight, relative to the total weight of the composition.

### Lipophilic gelling agents

Depending on the viscosity of the composition that it is desired to obtain, one or more gelling agents, which are hydrophilic, that is to say soluble or dispersible in the oily phase, may be incorporated into a composition of the invention.

Preferably, the lipophilic gelling agents will be chosen from natural gelling agents or gelling agents of natural origin.

Mention may be made, for example, as lipophilic gelling agents, of modified clays, such as modified magnesium silicate (Bentone Gel VS38^{®} from Rheox), hectorite modified with distearyldimethylammonium chloride (CTFA name: Disteardimonium Hectorite), such as the product sold under the name "Bentone 38 CE^{®}" by Rheox.

According to a particularly preferred embodiment, the composition of the invention in the form of a water-in-oil emulsion comprises, in particular in a physiologically acceptable medium:
a) a continuous oily phase comprising at least:
   (i) a mixture of undecane and tridecane; and
   (ii) macadamia oil and/or olive oil; and
b) an aqueous phase dispersed in said oily phase comprising:
   i) tannic acid; and
   ii) a pullulan; and
c) the surfactant polyglyceryl-6 polyricinoleate; and
d) at least 1,3-propanediol; and
e) at least glycerol.

According to a specific embodiment, said composition additionally comprises f) at least one sugar alcohol and more particularly sorbitol.

According to a specific embodiment, said composition additionally comprises g) at least one monoester of saturated and linear C₇-C₁₄ carboxylic acid and of glycerol and more particularly glyceryl caprylate.

According to a specific embodiment, said composition additionally comprises h) at least one pigment chosen from iron oxides, titanium dioxides and mixtures thereof; or chosen from iron oxides coated with at least one lipophilic or hydrophobic compound, titanium dioxides coated with at least one lipophilic or hydrophobic compound; and mixtures thereof.

According to a specific embodiment, said composition additionally comprises i) at least one inorganic filler more particularly chosen from natural or synthetic micas, calcium carbonate and mixtures thereof.

According to a specific embodiment, said composition additionally comprises j) at least one hydrophilic gelling agent which is natural or of natural origin and/or at least one lipophilic gelling agent which is natural or of natural origin, and more preferentially at least one lipophilic gelling agent chosen from modified clays, and more particularly a hectorite modified with distearyldimethylammonium chloride (INCI name: Disteardimonium Hectorite), such as the product sold under the name "Bentone 38 CE^{®}" by Rheox or the product "Bentone 38VCG" from Elementis.

The present invention also relates to a cosmetic composition comprising, in a physiologically acceptable medium, a composition as defined above.

The physiologically acceptable medium is generally adapted to the nature of the support onto which the composition has to be applied, and also to the appearance under which the composition has to be packaged.

The compositions according to the invention may also comprise additives commonly used in care and/or makeup products, such as active agents such as vitamins, for example vitamins A, E, C, B3, sunscreens, fillers, antioxidants, chelating agents, fragrances, neutralizing agents, preserving agents, and mixtures thereof.

It is a matter of routine operations for those skilled in the art to adjust the nature and the amount of the additives present in the compositions in accordance with the invention so that the cosmetic properties desired for these are not thereby affected.

According to one embodiment, a composition of the invention can advantageously be provided in the form of a composition for caring for the skin, in particular of the body or of the face, especially of the face.

According to another embodiment, a composition of the invention can advantageously be provided in the form of a composition for making up keratin materials, in particular the skin of the body or of the face, in particular of the face.

Thus, according to a sub-mode of this embodiment, a composition of the invention can advantageously be provided in the form of a base composition for makeup.

A composition of the invention can advantageously be provided in the form of a foundation.

According to another sub-mode of this embodiment, a composition of the invention can advantageously be provided in the form of a composition for making up the skin and in particular the face. It can thus be an eyeshadow or a face powder.

Such compositions are in particular prepared according to the general knowledge of those skilled in the art.

### Packaging and applicators

The compositions according to the invention may be packaged in a container delimiting at least one compartment that comprises said composition, said container being closed by a closing member.

The container may be in any suitable form. It may notably be in the form of a bottle, a tube, a jar or a case.

The closing member may be in the form of a removable stopper, a lid or a cover, notably of the type including a body fixed to the container and a cap articulated on the body. It may also be in the form of a member for selectively closing the container, notably a pump, a valve or a flap valve.

The container may be combined with an applicator, notably in the form of a fine brush, as described, for example, in patent FR 2 722 380. The applicator may be in the form of a block of foam or of elastomer. The applicator may be free (sponge) or securely fastened to a rod borne by the closing member, as described, for example, in patent US 5 492 426. The applicator may be securely fastened to the container, as described, for example, in patent FR 2 761 959.

The product may be contained directly in the container, or indirectly.

The closing member may be coupled to the container by screwing. Alternatively, the coupling between the closing member and the container occurs other than by screwing, notably via a bayonet mechanism, by click-fastening or by gripping. The term "click-fastening" in particular means any system involving the crossing of a bead or cord of material by elastic deformation of a portion, notably of the closing member, followed by return to the elastically unconstrained position of said portion after the bead or cord has been crossed.

The container may be at least partially made of thermoplastic material. Examples of thermoplastic materials that may be mentioned include polypropylene and polyethylene.

The container may have rigid or deformable walls, notably in the form of a tube or a tube bottle.

The container may comprise means intended to bring about or facilitate the dispensing of the composition. By way of example, the container may have deformable walls so as to make the composition exit in response to excess pressure inside the container, which excess pressure is brought about by the elastic (or non-elastic) squeezing of the walls of the container.

The container may be equipped with a drainer positioned in the vicinity of the opening of the container. Such a drainer makes it possible to wipe the applicator and possibly the rod to which it may be securely fastened. Such a drainer is described, for example, in patent FR 2 792 618.

Throughout the description, including the claims, the term "including a" should be understood as being synonymous with "including at least one", unless otherwise specified.

The expressions "between ... and ...", and "ranging from ... to ..." should be understood as meaning limits included, unless otherwise specified.

The invention is illustrated in more detail by the examples and figures presented below. Unless otherwise indicated, the amounts indicated are expressed as mass percentages.

### Example 1 and Counterexample 1

Composition 1 according to the invention and Counterexample 1 outside the invention were prepared.

| **Phase** | **Ingredients** | **Ex1** | **Counterex. 1** |
|---|---|---|---|
| A1 | Polyglyceryl-6 Polyricinoleate (SY-Glyster CRS-75^{®} - Sakamoto Yakuhin Kogyo) | 3.0 | 3.0 |
| | Dicaprylyl Ether | 1.0 | 1.0 |
| | Undecane (and) Tridecane (Cetiol Ultimate^{®} - BASF) | 22.0 | 22.0 |
| A2 | Disteardimonium Hectorite (Bentone 38 CE^{®} -Rheox) | 1.2 | 1.2 |
| A3 | Macadamia Ternifolia Seed Oil | 1.0 | 1.0 |
| | Tocopherol | 0.7 | 0.7 |
| A4 | Calcium Carbonate | 5.0 | 5.0 |
| B1 | AQUA | qs 100 | qs 100 |
| | Glycerol | 5.0 | 5.0 |
| B2 | Pullulan | 2.2 | 2.2 |
| B3 | Hydrogenated Starch Hydrolysate | 4.0 | 4.0 |
| | Glyceryl Caprylate | 0.5 | 0.5 |
| | Propanediol | 5.0 | 5.0 |
| B4 | Tannic acid (Brewtan F^{®}-Anijomoto Omnichem NV) | 5.0 | 0 |
| B5 | Sodium Chloride | 0.2 | 0.2 |
| C | Denat. alcohol | 5.5 | 5.5 |
| D | Synthetic Fluorophlogopite | 1.3 | 1.3 |
| | CI 77891 (and) Disodium Stearoyl Glutamate (and) Aluminum Hydroxide | 8.7 | 8.7 |
| | CI 77499 (and) Disodium Stearoyl Glutamate (and) Aluminum Hydroxide | 0.2 | 0.2 |
| | CI 77491 (and) Disodium Stearoyl Glutamate (and) Aluminum Hydroxide | 0.5 | 0.5 |
| | CI 77492 (and) Disodium Stearoyl Glutamate (and) Aluminum Hydroxide | 2.1 | 2.1 |

### Protocol for preparation of the compositions

The compositions of the invention were produced by preparing a white base not containing pigments, to which a pigment preparation was added in a second step.

### Preparation of the white base

The oily phase A1 was introduced into the main beaker, which is placed on a hot plate at 60°C, with stirring using a Moritz-type homogenizer.

Once the temperature was at 60°C, the hectorite A2 was introduced with stirring for 15 minutes until a homogeneous mixture was obtained.

The mixture was subsequently cooled to ambient temperature and kept stirred, then the phase A3 was added with stirring for 5 minutes. Finally, the phase A4 was introduced while stirring for 10 minutes.

The pullulan B2 was added to the aqueous phase B1, the water of which was preheated to 95°C, with stirring using a Rayneri-type homogenizer, for 20 minutes. The phase B3 was subsequently added with stirring for 10 minutes and the phase B4 was added with stirring for 20 minutes. Finally, the phase B5 was added with stirring for 5 minutes.

The emulsion was formed at ambient temperature: the aqueous phase was poured into the fatty phase, placed in a bath of water and ice, while gradually increasing the stirring speed until a vortex was obtained. The formulating was carried out using a Mortiz-type homogenizer.

Stirring was maintained for 10 minutes. Subsequently, the alcohol (phase C) was introduced and the mixture was kept stirred for 5 minutes.

### Pigmentation of the white base

The white base was introduced into a beaker, placed in a bath of water and ice, and then the phase D was slowly added with stirring. The stirring was subsequently maintained for 20 minutes with a vortex. Formulating was carried out using a Rayneri-type homogenizer.

### Wear property test

### Protocol

The wear property was characterized by a transfer index, determined using a rubbing test, while dry, in the presence of sebum, and in the presence of water, described below.

A support (square of 40 mm x 40 mm) constituted of a layer of neoprene foam that is adhesive on one of its faces (sold under the name RE70X40 212B from Joint Technique Lyonnais Ind.) was prepared. An adhesive crown having an inside diameter of 24 mm and a thickness of about 250 µm was applied onto the non-adhesive face of the support. The composition was applied inside the crown and was then levelled off with a glass slide to obtain a deposit of the composition about 250 µm thick, and the support was then allowed to dry for 24 hours in an oven at 37°C.

The crown was then removed and a drop of 10 µl of artificial sebum was applied, at the centre, onto the supports intended to measure the rubbing resistance in the presence of sebum, said artificial sebum having the following composition:

| **Ingredient** | **Concentration** |
|---|---|
| Triisostearin | 28.7 |
| Hydrogenated Polyisobutene | 13.7 |
| Oleic acid | 28.0 |
| Oleyl erucate | 22.9 |
| Octyldodecanol | 6.7 |

All the supports were dried at ambient temperature for 45 minutes.

The support was then adhesively bonded, via its adhesive face, onto a 35 mm x 35 mm square base weight 14cm high and having a mass of about 1 kg. Represented on a sheet of a paper was a strip 3.5 cm wide and 20.75 cm long and represented in this strip were 5 boxes each having a length of 4.15 cm along the longitudinal axis of the strip. This sheet was placed on a spreading bench (BYK-Drive Auto Applicator^{®} from BYK Instruments), connected to a vacuum pump (DOA-P504-BN^{®} from Gast Manufacturing Co Ltd). The support, adhesively bonded to the weight, was then deposited on the first box of the strip and the spreader was turned on, so as to move the weight with the support in a rectilinear and even manner over the entire length of the strip. The speed at which the weight with the support is moved is about 2.5 cm/s. The trail of product deposited on the strip of paper was observed. A mark, from 0 to 5, is then assigned, 0 corresponding to no transfer of the composition, 3 to partial transfer, and 5 to significant transfer. The remaining composition on the support was also observed in order to see whether or not it was modified by the rubbing.

### Results

| **Test** | | **Ex1** | **Counterex1** |
|---|---|---|---|
| While dry | Transfer index | 0 | 3 |
| | Modification of the deposit | No | No |
| In the presence of sebum | Transfer index | 0 | 5 |
| | Modification of the deposit | No | Yes |

It was noted that the composition of Example 1 has transfer indices of 0, and the deposits were not modified, contrary to Counterexample 1, which showed high transfer indices, and of which the deposit in the presence of sebum was modified. In addition, Example 1 had a better wear property, whether while dry or in the presence of sebum, than Counterexample 1. It was therefore noted that the presence of polyphenol X, in combination with a non-ionic polysaccharide Y, promotes the wear property of the deposit obtained with the composition.

## Claims

1. Composition for caring for and/or making up keratin materials, in particular the skin, in the form of a water-in-oil emulsion comprising, in particular in a physiologically acceptable medium:
a) a continuous oily phase comprising:
i) at least one plant oil; and
ii) at least one volatile alkane; and
b) at least one aqueous phase dispersed in the oil phase, comprising:
i) at least one polyphenol X comprising at least two different phenol groups, and
ii) at least one non-ionic polysaccharide Y, and
iii) at least one polyol;
c) at least one polyglycerolated non-ionic, preferably non-silicone, surfactant with an HLB of less than or equal to 8.

2. Composition according to Claim 1, not containing any silicone.

3. Composition according to Claim 1 or 2, comprising an oily phase in a content varying from 5% to 95%, in particular from 10% to 80%, in particular from 15% to 70% and more particularly from 20% to 65% by weight, relative to the total weight of the composition.

4. Composition according to Claim 1 or 2, in which the non-volatile plant oil is chosen from macadamia oil, olive oil, and mixtures thereof.

5. Composition according to any one of the preceding claims, comprising from 0.5% to 50% by weight of non-volatile plant oil(s), in particular ranging from 1% to 25% by weight and more particularly ranging from 1% to 10% by weight, relative to the total weight of the composition.

6. Composition according to any one of the preceding claims, in which the volatile alkane is chosen from branched alkanes such as C₈-C₁₆ isoalkanes of petroleum origin, notably chosen from isododecane, isodecane, isohexadecane, and more particularly isododecane.

7. Composition according to any one of Claims 1 to 5, in which the volatile alkane is chosen from volatile linear C₆-C₁₄ alkanes, notably from n-hexane (C₆); n-heptane (C₇), n-octane (C₈), n-nonane (C₉), n-decane (C₁₀), n-undecane (C₁₁), n-dodecane (C₁₂), n-tridecane (C₁₃) and n-tetradecane (C₁₄), and mixtures thereof.

8. Composition according to Claim 7, in which the volatile linear alkane is a mixture of volatile linear C₉-C₁₂ alkanes with the INCI name: C9-12 Alkane.

9. Composition according to Claim 7, in which the volatile linear alkane is a mixture of n-undecane (C₁₁) and n-tridecane (C₁₃).

10. Composition according to any one of the preceding claims, in which the volatile alkane(s) are present in a concentration ranging from 0.5% to 90% by weight, in particular from 1% to 50% by weight and more particularly from 5% to 40% by weight, relative to the total weight of the composition.

11. Composition according to any one of the preceding claims, in which the oily phase also comprises at least one additional non-volatile hydrocarbon-based oil, in particular dicaprylyl ether.

12. Composition according to Claim 11, in which the additional non-volatile hydrocarbon-based oil(s) are present in a concentration of less than or equal to 50% by weight and more particularly ranging from 1% to 25% by weight, relative to the total weight of the composition.

13. Composition according to any one of the preceding claims, in which the ratio by weight of the total amount of non-volatile plant oil(s) to the total amount of volatile alkane(s) ranges from 1/40 to 1/1 and preferably from 1/20 to 1/5.

14. Composition according to any one of the preceding claims, in which the ratio by weight of the total amount of non-volatile plant oil(s) and of additional non-volatile hydrocarbon-based oil(s) to the total amount of volatile alkane(s) ranges from 1/8 to 1/1 and preferably from 1/4 to 1/1.

15. Composition according any one of the preceding claims, in which the polyphenol is chosen from catechin tannins, notably chosen from gallotannins and ellagitannins.

16. Composition according to any one of the preceding claims, in which the polyphenol X is tannic acid.

17. Composition according to any one of the preceding claims, comprising water in a content ranging from 5% to 80%, more particularly from 20% to 65% and even more preferably from 20% to 55% by weight, relative to the total weight of the composition.

18. Composition according to any one of the preceding claims, in which the polyphenol(s) X is (are) present in an amount greater than or equal to 0.8% by weight, preferably greater than or equal to 1.0% by weight and more particularly greater than or equal to 2.0% by weight relative to the total weight of the composition.

19. Composition according to any one of the preceding claims, in which the polyphenol(s) X is (are) present in an amount ranging from 1.0% to 30.0% by weight, and more particularly ranging from 2.0% to 30% by weight relative to the total weight of the composition.

20. Composition according to any one of the preceding claims, in which the non-ionic polysaccharide(s) Y are chosen from pullulans; celluloses such as cetylhydroxyethylcellulose; modified guar gums; and mixtures thereof.

21. Composition according to any one of the preceding claims, in which the non-ionic polysaccharide Y is an unmodified pullulan.

22. Composition according to any one of the preceding claims, in which the polyglycerolated emulsifying non-ionic surfactant with an HLB ≤ 8 is chosen from:
- esters of isostearic acid and of a polyglycerol containing from 2 to 10 mol of glycerol units, such as for example Polyglyceryl-4 Isostearate; Polyglyceryl-3 Diisostearate; Polyglyceryl-2 Diisostearate; Polyglyceryl-10 Isostearate; Polyglyceryl-2 Triisostearate ;
- esters of stearic acid and of a polyglycerol containing 2 or 3 mol of glycerol units, such as Polyglyceryl-2 Sesquistearate, Polyglyceryl-3 Distearate and Polyglyceryl-2 Stearate;
- esters of oleic acid and of a polyglycerol containing 2 to 3 mol of glycerol units, such as Polyglyceryl-2 Oleate, Polyglyceryl-3 Oleate, Polyglyceryl-2 Dioleate; Polyglyceryl-3 Dioleate;
- polyglyceryl polyricinoleate(s) containing from 3 to 6 mol of glycerol units, such as Polyglyceryl-3 Polyricinoleate; Polyglyceryl-5; Polyglyceryl-6 Polyricinoleate;
- mixtures thereof.

23. Composition according to any one of the preceding claims, in which the polyglycerolated emulsifying non-ionic surfactant with an HLB ≤ 8 is chosen from polyglyceryl polyricinoleate(s) containing from 3 to 6 mol of glycerol units, in particular Polyglyceryl-6 Polyricinoleate.

24. Composition according to any one of the preceding claims, comprising from 2% to 10% by weight of polyglycerolated non-ionic surfactant(s) with an HLB ≤ 8, in particular from 2% to 7% by weight, relative to the total weight of the composition.

25. Composition according to any one of the preceding claims, in which the mole ratio of the reactive hydroxyl (OH) groups of the polyphenol(s) X to the sum of the reactive hydroxyl groups of the non-ionic polysaccharide(s) Y and of the reactive polyglycerolated groups of the polyglycerolated non-ionic surfactant(s) with the polyphenol(s) X preferentially ranges from 1/3 to 20, more preferentially from 1/2 to 15 and more particularly from 3/4 to 3.

26. Composition according to any one of the preceding claims, in which the polyol is chosen from C₃-C₄ alkanediols, glycerol, and mixtures thereof, in particular chosen from propanediol, glycerol, and mixtures thereof.

27. Composition according to any one of the preceding claims, in which the polyol is a mixture of propanediol and glycerol.

28. Composition according to any one of the preceding claims, comprising from 0.1% to 25% by weight of total amount of polyol(s), in particular from 1% to 15% by weight and more particularly from 1% to 10% by weight, relative to the total weight of the composition.

29. Composition according to any one of the preceding claims, also comprising at least one sugar alcohol; preferably chosen from sorbitol.

30. Composition according to any one of the preceding claims, also comprising at least one monoester of saturated and linear C₇-C₁₄ carboxylic acid and of glycerol, preferably chosen from
- Glyceryl Heptanoate (C7);
- Glyceryl Caprylate (C8);
- Glyceryl Caprylate (C8)/Caprate (C10);
- Glyceryl Caprate (C10);
- Glyceryl Laurate (C12);
- Glyceryl Myristate (C14);
- mixtures thereof, and more particularly Glyceryl Caprylate.

31. Composition according to any one of the preceding claims, also comprising at least one monoalcohol comprising from 2 to 8 carbon atoms, and more particularly ethanol.

32. Composition according to any one of the preceding claims, additionally comprising at least one pulverulent colorant, preferably chosen from pigments, nacres and mixtures thereof; and more particularly a mineral pigment chosen from metal oxides, in particular titanium dioxides, iron oxides, which are or are not coated, and mixtures thereof.

33. Composition according to any one of the preceding claims, additionally comprising at least one filler, preferably at least one inorganic filler, more particularly chosen from natural or synthetic micas, calcium carbonate and mixtures thereof.

34. Composition according to any one of the preceding claims, also comprising at least one lipophilic gelling agent; said gelling agent preferably being natural or of natural origin, and more particularly chosen from modified clays.

35. Cosmetic process for coating, notably making up, keratin materials, in particular the skin such as the face, the hands, the eyelids, the cheeks, comprising at least: the step of applying a composition as defined in any one of the preceding claims, to said keratin materials.

## Patentansprüche

1. Zusammensetzung zum Pflegen und/oder Schminken von Keratinmaterialien, insbesondere der Haut, in Form einer Wasser-in-Öl-Emulsion, umfassend, insbesondere in einem physiologisch unbedenklichen Medium:
a) eine kontinuierliche ölige Phase, umfassend
i) mindestens ein Pflanzenöl und
ii) mindestens ein flüchtiges Alkan; und
b) mindestens eine in der Ölphase dispergierte wässrige Phase, umfassend:
i) mindestens ein Polyphenol X mit mindestens zwei verschiedenen Phenolgruppen und
ii) mindestens ein nichtionisches Polysaccharid Y und
iii) mindestens ein Polyol;
c) mindestens ein polyglyceriniertes nichtionisches Tensid, vorzugsweise Nichtsilikontensid, mit einem HLB-Wert kleiner oder gleich 8.

2. Zusammensetzung nach Anspruch 1, die kein Silikon enthält.

3. Zusammensetzung nach Anspruch 1 oder 2, umfassend eine ölige Phase in einem Gehalt von 5 bis 95 Gew.-%, insbesondere 10 bis 80 Gew.-%, insbesondere 15 bis 70 Gew.-% und spezieller 20 bis 65 Gew.-%, bezogen auf das Gesamtgewicht der Zusammensetzung.

4. Zusammensetzung nach Anspruch 1 oder 2, wobei das nichtflüchtige Pflanzenöl aus Macadamiaöl, Olivenöl und Mischungen davon ausgewählt ist.

5. Zusammensetzung nach einem der vorhergehenden Ansprüche, umfassend 0,5 bis 50 Gew.-% nichtflüchtiges Pflanzenöl bzw. nichtflüchtige Pflanzenöle, insbesondere im Bereich von 1 bis 25 Gew.-% und spezieller im Bereich von 1 bis 10 Gew.-%, bezogen auf das Gesamtgewicht der Zusammensetzung.

6. Zusammensetzung nach einem der vorhergehenden Ansprüche, wobei das flüchtige Alkan aus verzweigten Alkanen wie C₈-C₁₆ -Isoalkanen petrochemischen Ursprungs ausgewählt ist, insbesondere aus Isododecan, Isodecan, Isohexadecan und spezieller Isododecan ausgewählt ist.

7. Zusammensetzung nach einem der Ansprüche 1 bis 5, wobei das flüchtige Alkan aus flüchtigen linearen C₆-C₁₄-Alkanen ausgewählt ist, insbesondere aus n-Hexan (C₆), n-Heptan (C₇), n-Octan (C₈), n-Nonan (C₉), n-Decan (C₁₀), n-Undecan (C₁₁), n-Dodecan (C₁₂), n-Tridecan (C₁₃) und n-Tetradecan (C₁₄), und Mischungen davon ausgewählt ist.

8. Zusammensetzung nach Anspruch 7, wobei es sich bei dem flüchtigen linearen Alkan um eine Mischung von flüchtigen linearen C₉-C₁₂-Alkanen mit dem folgenden INCI-Namen handelt: C9-12 Alkane.

9. Zusammensetzung nach Anspruch 7, wobei es sich bei dem flüchtigen linearen Alkan um eine Mischung von n-Undecan (C₁₁) und n-Tridecan (C₁₃) handelt.

10. Zusammensetzung nach einem der vorhergehenden Ansprüche, wobei das flüchtige Alkan bzw. die flüchtigen Alkane in einer Konzentration im Bereich von 0,5 bis 90 Gew.-%, insbesondere von 1 bis 50 Gew.-% und spezieller von 5 bis 40 Gew.-%, bezogen auf das Gesamtgewicht der Zusammensetzung, vorliegt bzw. vorliegen.

11. Zusammensetzung nach einem der vorhergehenden Ansprüche, wobei die ölige Phase außerdem mindestens ein zusätzliches nichtflüchtiges Öl auf Kohlenwasserstoffbasis, insbesondere Dicaprylylether, umfasst.

12. Zusammensetzung nach Anspruch 11, wobei das zusätzliche nichtflüchtige Öl bzw. die zusätzlichen nichtflüchtigen Öle auf Kohlenwasserstoffbasis in einer Konzentration von weniger oder gleich 50 Gew.-% und spezieller im Bereich von 1 bis 25 Gew.-%, bezogen auf das Gesamtgewicht der Zusammensetzung, vorliegt bzw. vorliegen.

13. Zusammensetzung nach einem der vorhergehenden Ansprüche, wobei das Gewichtsverhältnis der Gesamtmenge von nichtflüchtigem Pflanzenöl bzw. nichtflüchtigen Pflanzenölen zur Gesamtmenge von flüchtigem Alkan bzw. flüchtigen Alkanen im Bereich von 1/40 bis 1/1 und vorzugsweise von 1/20 bis 1/5 liegt.

14. Zusammensetzung nach einem der vorhergehenden Ansprüche, wobei das Gewichtsverhältnis der Gesamtmenge von nichtflüchtigem Pflanzenöl bzw. nichtflüchtigen Pflanzenölen und zusätzlichem nichtflüchtigen Öl bzw. zusätzlichen nichtflüchtigen Ölen auf Kohlenwasserstoffbasis zur Gesamtmenge von flüchtigem Alkan bzw. flüchtigen Alkanen im Bereich von 1/8 bis 1/1 und vorzugsweise von 1/4 bis 1/1 liegt.

15. Zusammensetzung nach einem der vorhergehenden Ansprüche, wobei das Polyphenol aus Catechintanninen ausgewählt ist und insbesondere aus Gallotanninen und Ellagitanninen ausgewählt ist.

16. Zusammensetzung nach einem der vorhergehenden Ansprüche, wobei es sich bei dem Polyphenol X um Tanninsäure handelt.

17. Zusammensetzung nach einem der vorhergehenden Ansprüche, umfassend Wasser in einem Gehalt im Bereich von 5 bis 80 Gew.-%, spezieller von 20 bis 65 Gew.-% und noch weiter bevorzugt von 20 bis 55 Gew.-%, bezogen auf das Gesamtgewicht der Zusammensetzung.

18. Zusammensetzung nach einem der vorhergehenden Ansprüche, wobei das Polyphenol bzw. die Polyphenole X in einer Menge größer oder gleich 0,8 Gew.-%, vorzugsweise größer oder gleich 1,0 Gew.-% und spezieller größer oder gleich 2,0 Gew.-%, bezogen auf das Gesamtgewicht der Zusammensetzung, vorliegt bzw. vorliegen.

19. Zusammensetzung nach einem der vorhergehenden Ansprüche, wobei das Polyphenol bzw. die Polyphenole X in einer Menge im Bereich von 1,0 bis 30,0 Gew.-% und spezieller im Bereich von 2,0 bis 30 Gew.-%, bezogen auf das Gesamtgewicht der Zusammensetzung, vorliegt bzw. vorliegen.

20. Zusammensetzung nach einem der vorhergehenden Ansprüche, wobei das nichtionische Polysaccharid bzw. die nichtionischen Polysaccharide Y aus Pullulanen; Cellulosen wie Cetylhydroxyethylcellulose; modifizierten Guargummen und Mischungen davon ausgewählt sind.

21. Zusammensetzung nach einem der vorhergehenden Ansprüche, wobei es sich bei dem nichtionischen Polysaccharid Y um unmodifiziertes Pullulan handelt.

22. Zusammensetzung nach einem der vorhergehenden Ansprüche, wobei das polyglycerinierte emulgierende nichtionische Tensid mit einem HLB-Wert ≤ 8 ausgewählt ist aus:
- Estern von Isostearinsäure und einem Polyglycerin mit 2 bis 10 mol Glycerineinheiten wie beispielsweise Polyglyceryl-4-isostearat; Polyglyceryl-3-diisostearat; Polyglyceryl-2-diisostearat; Polyglyceryl-10-isostearat; Polyglyceryl-2-triisostearat;
- Estern von Stearinsäure und einem Polyglycerin mit 2 oder 3 mol Glycerineinheiten, wie Polyglyceryl-2-sesquistearat, Polyglyceryl-3-distearat und Polyglyceryl-2-stearat;
- Estern von Ölsäure und einem Polyglycerin mit 2 bis 3 mol Glycerineinheiten, wie Polyglyceryl-2-oleat, Polyglyceryl-3-oleat, Polyglyceryl-2-dioleat; Polyglyceryl-3-dioleat;
- Polyglycerylpolyricinoleat (en) mit 3 bis 6 mol Glycerineinheiten, wie Polyglyceryl-3-polyricinoleat; Polyglyceryl-5-; Polyglyceryl-6-polyricinoleat;
- Mischungen davon.

23. Zusammensetzung nach einem der vorhergehenden Ansprüche, wobei das polyglycerinierte emulgierende nichtionische Tensid mit einem HLB-Wert ≤ 8 aus Polyglycerylpolyricinoleat(en) mit 3 bis 6 mol Glycerineinheiten, insbesondere Polyglyceryl-6-polyricinoleat, ausgewählt ist.

24. Zusammensetzung nach einem der vorhergehenden Ansprüche, umfassend 2 bis 10 Gew.-% polyglyceriniertes nichtionisches Tensid bzw. polyglycerinierte nichtionische Tenside mit einem HLB-Wert ≤ 8, insbesondere 2 bis 7 Gew.-%, bezogen auf das Gesamtgewicht der Zusammensetzung.

25. Zusammensetzung nach einem der vorhergehenden Ansprüche, wobei das Molverhältnis der reaktiven Hydroxylgruppen (OH-Gruppen) des Polyphenols bzw. der Polyphenole X zur Summe der reaktiven Hydroxylgruppen des nichtionischen Polysaccharids bzw. der nichtionischen Polysaccharide Y und der reaktiven polyglycerinierten Gruppen des polyglycerinierten nichtionischen Tensids bzw. der polyglycerinierten nichtionischen Tenside mit dem Polyphenol bzw. den Polyphenolen X bevorzugt im Bereich von 1/3 bis 20, weiter bevorzugt von 1/2 bis 15 und spezieller von 3/4 bis 3 liegt.

26. Zusammensetzung nach einem der vorhergehenden Ansprüche, wobei das Polyol aus C₃-C₄-Alkandiolen, Glycerin und Mischungen davon ausgewählt ist und insbesondere aus Propandiol, Glycerin und Mischungen davon ausgewählt ist.

27. Zusammensetzung nach einem der vorhergehenden Ansprüche, wobei es sich bei dem Polyol um eine Mischung von Propandiol und Glycerin handelt.

28. Zusammensetzung nach einem der vorhergehenden Ansprüche, umfassend 0,1 bis 25 Gew.-% der Gesamtmenge an Polyol bzw. Polyolen, insbesondere 1 bis 15 Gew.-% und spezieller von 1 bis 10 Gew.-%, bezogen auf das Gesamtgewicht der Zusammensetzung.

29. Zusammensetzung nach einem der vorhergehenden Ansprüche, außerdem umfassend mindestens einen Zuckeralkohol, der vorzugsweise aus Sorbitol ausgewählt ist.

30. Zusammensetzung nach einem der vorhergehenden Ansprüche, außerdem umfassend mindestens einen Monoester von einer gesättigten und linearen C₇-C₁₄-Carbonsäure und Glycerin, vorzugsweise ausgewählt aus
- Glycerylheptanoat (C7);
- Glycerylcaprylat (C8);
- Glycerylcaprylat (C8)/-caprat (C10);
- Glycerylcaprat (C10);
- Glyceryllaurat (C12);
- Glycerylmyristat (C14);
- Mischungen davon, insbesondere Glycerylcaprylat.

31. Zusammensetzung nach einem der vorhergehenden Ansprüche, die außerdem mindestens einen Monoalkohol mit 2 bis 8 Kohlenstoffatomen und spezieller Ethanol umfasst.

32. Zusammensetzung nach einem der vorhergehenden Ansprüche, zusätzlich umfassend mindestens ein pulverförmiges Farbmittel, das vorzugsweise aus Pigmenten, Perlglanzmitteln und Mischungen davon ausgewählt ist; und spezieller ein mineralisches Pigment, das aus Metalloxiden, insbesondere Titandioxiden, Eisenoxiden, die gegebenenfalls beschichtet sind, und Mischungen davon ausgewählt ist.

33. Zusammensetzung nach einem der vorhergehenden Ansprüche, zusätzlich umfassend mindestens einen Füllstoff, vorzugsweise mindestens einen anorganischen Füllstoff, der spezieller aus natürlichen oder synthetischen Glimmern, Calciumcarbonat und Mischungen davon ausgewählt ist.

34. Zusammensetzung nach einem der vorhergehenden Ansprüche, außerdem umfassend mindestens ein lipophiles Geliermittel; wobei das Geliermittel vorzugsweise natürlich oder natürlichen Ursprungs ist und insbesondere aus modifizierten Tonen ausgewählt ist.

35. Kosmetisches Verfahren zum Beschichten, insbesondere Schminken, von Keratinmaterialien, insbesondere der Haut wie dem Gesicht, den Händen, den Augenlidern, den Wangen, umfassend mindestens: den Schritt des Aufbringens einer Zusammensetzung gemäß einem der vorhergehenden Ansprüche auf die Keratinmaterialien.

## Revendications

1. Composition d'entretien et/ou de maquillage de matières kératiniques, en particulier de la peau, sous la forme d'une émulsion eau-dans-huile comprenant, en particulier dans un milieu physiologiquement acceptable :
a) une phase huileuse continue comprenant :
i) au moins une huile végétale ; et
ii) au moins un alcane volatil ; et
b) au moins une phase aqueuse dispersée dans la phase huileuse, comprenant :
i) au moins un polyphénol X comprenant au moins deux groupes phénol différents, et
ii) au moins un polysaccharide non ionique Y, et
iii) au moins un polyol ;
c) au moins un tensioactif non ionique polyglycérolé, de préférence non de silicone ayant un HLB inférieur ou égal à 8.

2. Composition selon la revendication 1 ne contenant pas une quelconque silicone.

3. Composition selon la revendication 1 ou 2, comprenant une phase huileuse en une teneur variant de 5 % à 95 %, en particulier de 10 % à 80 %, en particulier de 15 % à 70 %, et plus particulièrement de 20 % à 65 % en poids, par rapport au poids total de la composition.

4. Composition selon la revendication 1 ou 2 dans laquelle l'huile végétale non volatile est choisie parmi l'huile de macadamia, l'huile d'olive et leurs mélanges.

5. Composition selon l'une quelconque des revendications précédentes, comprenant de 0,5 % à 50 % en poids d'une ou plusieurs huiles végétales non volatiles, en particulier dans la plage de 1 % à 25 % en poids et plus particulièrement dans la plage de 1 % à 10 % en poids, par rapport au poids total de la composition.

6. Composition selon l'une quelconque des revendications précédentes, dans laquelle l'alcane volatil est choisi parmi les alcanes ramifiés comme les isoalcanes en C₈-C₁₆ d'origine pétrolière, notamment choisis parmi l'isododécane, l'isodécane, l'isohexadécane, et plus particulièrement l'isododécane.

7. Composition selon l'une quelconque des revendications 1 à 5, dans laquelle l'alcane volatil est choisi parmi les alcanes linéaires volatils en C₆-C₁₄, notamment parmi le n-hexane (C₆) ; le n-heptane (C₇), le n-octane (C₈), le n-nonane (C₉), le n-décane (C₁₀), le n-undécane (C₁₁), le n-dodécane (C₁₂), le n-tridécane (C₁₃), le n-tétradécane (C₁₄), et leurs mélanges.

8. Composition selon la revendication 7, dans laquelle l'alcane linéaire volatil est un mélange d'alcanes linéaires volatils en C₉-C₁₂ de nom INCI : C9-12 Alkane.

9. Composition selon la revendication 7, dans laquelle l'alcane linéaire volatil est un mélange de n-undécane (C₁₁) et de n-tridécane (C₁₃) .

10. Composition selon l'une quelconque des revendications précédentes, dans laquelle l'alcane ou les alcanes non volatils sont présents en une concentration dans la plage de 0,5 % à 90 % en poids, en particulier de 1 % à 50 % en poids et plus particulièrement de 5 % à 40 % en poids par rapport au poids total de la composition.

11. Composition selon l'une quelconque des revendications précédentes, dans laquelle la phase huileuse comprend en outre au moins une huile à base d'hydrocarbure non volatile supplémentaire, en particulier l'éther de dicaprylyle.

12. Composition selon la revendication 11, dans laquelle l'huile ou les huiles à base d'hydrocarbure non volatiles sont présentes en une concentration inférieure ou égale à 50 % en poids et plus particulièrement dans la plage de 1 % à 25 % en poids, par rapport au poids total de la composition.

13. Composition selon l'une quelconque des revendications précédentes, dans laquelle le rapport pondéral de la quantité totale d'huile(s) végétale(s) non volatile(s) sur la quantité totale d'alcane(s) volatil(s) va de 1/40 à 1/1, et de préférence de 1/20 à 1/5.

14. Composition selon l'une quelconque des revendications précédentes, dans laquelle le rapport pondéral de la quantité totale d'huile(s) végétale(s) non volatile(s) et d'huile(s) à base d'hydrocarbure non volatile(s) supplémentaire(s) sur la quantité totale d'alcane(s) volatil(s) va de 1/8 à 1/1, et de préférence de 1/4 à 1/1.

15. Composition selon l'une quelconque des revendications précédentes, dans laquelle le polyphénol est choisi parmi les tanins de catéchine, notamment choisi parmi les gallotanins et les ellagitanins.

16. Composition selon l'une quelconque des revendications précédentes, dans laquelle le polyphénol X est l'acide tannique.

17. Composition selon l'une quelconque des revendications précédentes, comprenant de l'eau en une teneur dans la plage de 5 % à 80 %, plus particulièrement de 20 % à 65 % et encore plus préférablement de 20 % à 55 % en poids par rapport au poids total de la composition.

18. Composition selon l'une quelconque des revendications précédentes, dans laquelle le ou les polyphénol(s) X est(sont) présent(s) en une quantité supérieure ou égale à 0,8 % en poids, de préférence supérieure ou égale à 1,0 % en poids, et plus particulièrement supérieure ou égale à 2,0 % en poids par rapport au poids total de la composition.

19. Composition selon l'une quelconque des revendications précédentes, dans laquelle le ou les polyphénol(s) X est(sont) présent(s) en une quantité allant de 1,0 % à 30,0 % en poids, et plus particulièrement allant de 2,0 % à 30 % en poids par rapport au poids total de la composition.

20. Composition selon l'une quelconque des revendications précédentes, dans laquelle le ou les polysaccharide(s) non ionique(s) Y est(sont) choisi(s) parmi les pullulanes ; les celluloses telles que la cétylhydroxyéthylcellulose ; les gommes de guar modifiées ; et leurs mélanges.

21. Composition selon l'une quelconque des revendications précédentes, dans laquelle le polysaccharide non ionique Y est un pullulane non modifié.

22. Composition selon l'une quelconque des revendications précédentes, dans laquelle le tensioactif non ionique émulsifiant polyglycérolé ayant un HLB ≤ 8 est choisi parmi :
- les esters d'acide isostéarique et d'un polyglycérol ayant de 2 à 10 moles de motifs de glycérol, tels que par exemple Polyglyceryl-4 Isostearate; Polyglyceryl-3 Diisostearate; Polyglyceryl-2 Diisostearate; Polyglyceryl-10 Isostearate; Polyglyceryl-2 Triisostearate ;
- les esters d'acide stéarique et d'un polyglycérol contenant 2 ou 3 moles de motifs de glycérol, tels que le Polyglyceryl-2 Sesquistearate, Polyglyceryl-3 Distearate et Polyglyceryl-2 Stearate;
- les esters d'acide oléique et d'un polyglycérol contenant 2 à 3 moles de motifs de glycérol, tels que Polyglyceryl-2 Oleate, Polyglyceryl-3 Oleate, Polyglyceryl-2 Dioleate; Polyglyceryl-3 Dioleate;
- un ou plusieurs polyricinoléate(s) de polyglycéryle contenant de 3 à 6 moles de motifs de glycérol, tels que Polyglyceryl-3 Polyricinoleate; Polyglyceryl-5; Polyglyceryl-6 Polyricinoleate;
- leurs mélanges.

23. Composition selon l'une quelconque des revendications précédentes, dans laquelle le tensioactif non ionique émulsifiant polyglycérolé ayant un HLB ≤ 8 est choisi parmi un ou plusieurs polyricinoléate(s) de polyglycéryle ayant de 3 à 6 moles de motifs de glycérol, en particulier Polyglyceryl-6 Polyricinoleate.

24. Composition selon l'une quelconque des revendications précédentes, comprenant de 2 à 10 % en poids de tensioactif(s) non ionique(s) polyglycérolé(s) ayant un HLB ≤ 8, en particulier de 2 à 7 % en poids, par rapport au poids total de la composition.

25. Composition selon l'une quelconque des revendications précédentes, dans laquelle le rapport molaire des groupes hydroxyle réactifs (OH) du ou des polyphénol(s) X sur la somme des groupes hydroxyle réactifs du ou des polysaccharide(s) non ionique(s) Y et des groupes polyglycérolés réactifs du ou des tensioactif(s) non ionique(s) polyglycérolé(s) avec le ou les polyphénol(s) X va préférentiellement de 1/3 à 20, plus préférentiellement de 1/2 à 15, et plus particulièrement de 3/4 à 3.

26. Composition selon l'une quelconque des revendications précédentes, dans laquelle le polyol est choisi parmi les alcanediols en C₃-C₄, le glycérol , et leurs mélanges, en particulier choisi parmi le propanediol, le glycérol, et leurs mélanges.

27. Composition selon l'une quelconque des revendications précédentes dans laquelle le polyol est un mélange de propanediol et de glycérol.

28. Composition selon l'une quelconque des revendications précédentes, comprenant de 0,1 % à 25 % en poids de quantité totale de polyol(s), en particulier de 1 % à 15 % en poids et plus particulièrement de 1 % à 10 % en poids, par rapport au poids total de la composition.

29. Composition selon l'une quelconque des revendications précédentes, comprenant également au moins un alcool de sucre ; de préférence choisi parmi le sorbitol.

30. Composition selon l'une quelconque des revendications précédentes, comprenant en outre au moins un monoester d'acide carboxylique en C₇-C₁₄, saturé et linéaire et de glycérol, de préférence choisi parmi
- Glyceryl Heptanoate (C7);
- Glyceryl Caprylate (C8);
- Glyceryl Caprylate (C8)/Caprate (C10);
- Glyceryl Caprate (C10);
- Glyceryl Laurate (C12);
- Glyceryl Myristate (C14);
- leurs mélanges, et plus particulièrement Glyceryl Caprylate.

31. Composition selon l'une quelconque des revendications précédentes comprenant en outre au moins un monoalcool comprenant de 2 à 8 atomes de carbone, et plus particulièrement l'éthanol.

32. Composition selon l'une quelconque des revendications précédentes, comprenant en outre au moins une matière colorante pulvérulente, de préférence choisie parmi les pigments, les nacres et leurs mélanges ; et plus particulièrement un pigment minéral choisi parmi les oxydes métalliques, en particulier les dioxydes de titane, les oxydes de fer, qui sont revêtus ou non, et leurs mélanges.

33. Composition selon l'une quelconque des revendications précédentes, comprenant en outre au moins une charge, de préférence au moins une charge minérale, plus particulièrement choisie parmi les micas naturels ou synthétiques, le carbonate de calcium et leurs mélanges.

34. Composition selon l'une quelconque des revendications précédentes, comprenant en outre au moins un agent gélifiant lipophile ; de préférence ledit agent gélifiant étant naturel ou d'origine naturelle, et plus particulièrement choisi parmi les argiles modifiées.

35. Procédé cosmétique pour le revêtement, notamment le maquillage, de matières kératiniques, en particulier la peau comme le visage, les mains, les paupières, les joues, comprenant au moins : l'étape d'application d'une composition telle que définie dans l'une quelconque des revendications précédentes, sur lesdites matières kératiniques.
